# EUROPEAN PATENT SPECIFICATION

(11) **EP 1 704 237 B1**
(45) Date of publication and mention of the grant of the patent: **04.04.2012**
(21) Application number: 05707054.2
(22) Date of filing: 14.01.2005
(51) Int. Cl.: C12N 15/54, C12N 9/12, C12N 15/82, A01H 5/00, A01H 17/00

(54) **CCAMK INVOLVED IN NODULATION AND ENDOMYCORRHIZATION**
AN DER BILDUNG VON KNÖLLCHEN UND ENDOMYKORRHIZA BETEILIGTE CCAMK
CAM KINASES INTERVENANT DANS LA NODULATION ET L'ENDOMYCORHIZATION

(30) Priority: 15.01.2004 EP 04290107
(43) Date of publication of application: 27.09.2006
(73) Proprietor: INSTITUT NATIONAL DE LA RECHERCHE AGRONOMIQUE (INRA), 75007 Paris Cedex 07 (FR)
(72) Inventor: DEBELLE, Frédéric, F-31400 Toulouse (FR); LEVY, Julien, F-31000 Toulouse (FR); BRES, Cécile, F-33650 La Brede (FR); ROSENBERG, Charles, F-31320 Castanet Tolosan (FR)
(74) Representative: Vialle-Presles, Marie José
(86) International application number: PCT/EP2005/000832
(87) International publication number: WO 2005/068636

(56) References cited:
- WO-A-97/26363
- WO-A-97/35968
- PANDEY ET AL.: "A Ca2+/CaM-dependent kinase from pea is stress regulated and in vitro phosphorylates a protein that binds to AtCaM5 promoter" EUR. J. BIOCHEM., vol. 269, 2002, pages 3193-3204, XP002278188
- DATABASE EM_EST [Online] EMBL; 12 April 2001 (2001-04-12), FEDOROVA ET AL.: "EST482881 GVN Medicago truncatula cDNA clone pGVN-63N24 5' end, mRNA sequence" XP002278191 retrieved from EBI Database accession no. BG581151
- DATABASE EM_GSS [Online] EMBL; 16 December 2003 (2003-12-16), TOWN ET AL.: "MBEGB88TF mth2 Medicago truncatula genomic clone 4808, genomic survey sequence" XP002278192 retrieved from EBI Database accession no. CG951356
- DATABASE EM_EST [Online] EMBL; 21 December 2000 (2000-12-21), KORTH ET AL.: "NF034F02IN1F1026 Insect herbivory Medicago Truncatula cDNA clone NF034F02IN 5', mRNA sequence" XP002278193 retrieved from EBI Database accession no. BF641014
- CATOIRA ET AL.: "Four genes of Medicago truncatula controlling components of a Nod factor transduction pathway" THE PLANT CELL, vol. 12, September 2000 (2000-09), pages 1647-1665, XP002278189 cited in the application
- SHAW ET AL.: "Nod factor elicits two separable calcium responses in Medicago truncatula root hair cells" PLANT PHYSIOLOGY, vol. 131, March 2003 (2003-03), pages 976-984, XP002278190 cited in the application
- ENDRE GABRIELLA ET AL: "A receptor kinase gene regulating symbiotic nodule development" NATURE, MACMILLAN JOURNALS LTD. LONDON, GB, vol. 417, no. 6892, 27 June 2002 (2002-06-27), pages 962-966, XP002245535 ISSN: 0028-0836 cited in the application

### SEQUENCE LISTING

<110> INSTITUT NATIONAL DE LA RECHERCHE AGRONOMIQUE
   DEBELLE, Frédéric
   LEVY, Julien
   BRES, Cécile
   ROSENBERG, Charles
<120> CCaMK involved in nodulation and endomycorrhization
<130> MJPbv539/122
<150> EP04290107.4
   <151> 2004/01/15
<160> 43
<170> PatentIn version 3.1
<210> 1
   <211> 11200
   <212> DNA
   <213> Medicago truncatula
<220>
   <221> exon
   <222> (3171)..(3901)
   <223>
<220>
   <221> exon
   <222> (4467) .. (4521)
   <223>
<220>
   <221> exon
   <222> (4600)..(4710)
   <223>
<220>
   <221> exon
   <222> (5000) .. (5232)
   <223>
<220>
   <221> exon
   <222> (5764) .. (5971)
   <223>
<220>
   <221> exon
   <222> (6627)..(6686)
   <223>
<220>
   <221> exon
   <222> (6769)..(6942)
   <223>
<400> 1
   ggcacagccg ttacacatgg ggtcgggtgt ttcaatggag attccgtttg cagatagtca      60
   tatggcattg gatacgcaga tgcctggaag aaaaaggagc actccggagg atatggtgga     120
   gaagactgtt gagagaaggc agaaaaggat gatcaagaat cgggaatctg ctgcccgttc     180
   aagagcacgg aaacaggtga tctctctctt tctttatttc aagtgttgta actcgcaagt     240
   ccttattatt tgattggttt ttgttgtaat gaatacccta aacctgaata ataactttaa     300
   caagcaggct tacacaaatg aattggagat taaagtttca cgcctggaag aggaaaatga     360
   aatgctgagg aaacgaaagg tttgattctt ctttgaaatt ataagtttaa ttgatttgac     420
   tgagtatgtt tttataatga tcctaattcc tttcattccc cagattgagt agtttgtaca     480
   tttgtggatt ataatttcag cataagatag gagacagtct cagtttcaat tactatacaa     540
   atctctgcat ttttttggat taatgaaact ctgcatatta tgttctctag ttgagacctg     600
   agcatgtgtc atttgcattt gttaaatttg aattaaagag gaatcggtcc attgatcaga     660
   agcaagtctt gtaacgtaga ataaacattg ttaaatcatt acaccatcag cattgccgtc     720
   ttgatgttat ttatttggat gtatctctct tatggcctct gctctatctt ttacatagtc     780
   attaagaatt gttcaggaaa ataaatcaca tgagacacat ttaattttgt gattgacaaa     840
   cacaaatgaa catgttagtt ctgatgcaac ttttggatta ttctgctctg aatataccac     900
   aatgaatctc actaagggga tttaaatttt tccattcata caaaaacctt accgtgtgac     960
   atgtttccca cttagagcac agagttgcac ctttgaaata aaaattactg ctttcattaa   1020
   ttgagtttca tatcaggaac tatatgcctt gaatttccac tgcttcgtta agtcagtttt   1080
   ttttatattg ggtgatttgg aatttgattt atcggctcaa cagtctttgc tctttttcat   1140
   tcaattagca gtgcttgtgg aagatcttgg atcgtacaac catacatgct ataataaaaa   1200
   ctatatctat atgcacattg tatccatggt catttttacc tgttaaaatc atgggaacaa   1260
   aattgctctt ttatacatgc taacttactc ctttgccttg tatatactat cagattatat   1320
   tattaatttt tgtcctcaac atgcaggagc tagagaatat gttgccttgt gcaccgattg   1380
   ctgaacctaa atatcagcta cgccgaatag catcctgtcc attctgagat ctttgtacct   1440
   ataagtagag gtttgtagat atgaaaatca gggaatgttt tcttgtagtt caatgtttgg   1500
   ctgacacctg ccttttgtta tctctggaat actctgttaa gttcttaagt aattatagct   1560
   aatgttgtag ttttattatt cattacatag ccattttaat tattggttaa tttcatcttg   1620
   gtctattggc gatagaataa tagtcgggtc ttgccaattt cgttgtcatg agaaaatagg   1680
   accactttta gcagagagtt gttattcaca cccctgtttt tctaagacat ttccctaacg   1740
   agttctttca aaatttgccc aaaatttcct gtatggaagt accttctggg gaatatattc   1800
   ccatttctta cttattatac cttctgaaag tgtgctttca gagtatttat aactttcaga   1860
   caattctagt gcatatcttt tatacatttt ggaggttgat tgtgtggatg caatggagta   1920
   tttgaagaaa ttagtacatt aatcatttga tagagttttc tgatacatga ttgcattttg   1980
   cccctttgct agttgcttgg tatacactct aaactctagc agtcccttaa ccttcacgat   2040
   ggcccctata atgagtagta taagtgcaaa tgtataatgg gcatatcctc tgatggcatc   2100
   ttccaccatt tgttgatcaa agtctagatg tgtgccattc acgatggccc ctatttgaat   2160
   tttatcttta tatttattgc agaagaaaac acattgttat atatttacca aaaatattta   2220
   tttaatagta aaaaatttac gaaaaccaag aaccagtctt tgtgatggca atttctttta   2280
   ttttatggaa tagcaaatcc atatcttgat gcgcacgcgc gtattataaa caataataat   2340
   actcttttta tttttcaaaa aaaatttttt tttagagcaa acaataataa tactttacaa   2400
   tttctttttc cttaaaaaag caataatact ttacaaaaaa aaaaaaaaaa aacactcttg   2460
   ttttccgcta aaaaaataac actttttacg agaacaaaac tatatgtaat ttcttctcta   2520
   agaaaaacta tatatggttt ttctcctaaa aaaactatat ataatatctg taatagaaaa   2580
   agctatatat ttcaaagaaa atcaaacaaa tatactctgt ggaatggaat gatgaattaa   2640
   ggggttgtta gacaataatc gagtcaccta aaaattattt tttttatata caaagagaaa   2700
   ggaaaaaaaa aacttatcat gatatgcatt ttgttaaaaa taaaaaccaa aattcagttt   2760
   tcttagtgtt actcttttgt tttatacttt agccagaaac acatttctaa tttcaatttg   2820
   gccaaatgtt gaacagtgaa agttagaata caaatgtagc agttgactct ctctttgtcc   2880
   attctaaaac agctcttacc agcctactta atattccctt gtacctatct taatttaatt   2940
   catattttat ttcataaaca aatacaataa ctcttatcct tcaaaaataa aaaatacaat   3000
   aactcttata ttattatatt tatatgctaa tgtcaatttc aaccatttca ataacgcgtt   3060
   ttatatgctc tcattattga acatattata taccatcttg ttctttttct atagttcctt   3120
   gtctttgcac attttctaag tcttcaaaaa tattccattt tttcatcacc atg gga      3176
                                                          Met Gly
   1
   tat gga aca aga aaa ctc tca gat gaa tat gaa gtt tca gaa att cta 3224
   Tyr Gly Thr Arg Lys Leu Ser Asp Glu Tyr Glu Val Ser Glu Ile Leu
   5 10 15
   ggt aga ggt gga ttt tct gtt gtt aga aaa ggt aca aaa aaa tca agc 3272
   Gly Arg Gly Gly Phe Ser Val Val Arg Lys Gly Thr Lys Lys Ser Ser
   20 25 30
   att gaa gaa gaa aaa tca caa tca caa gta gca atc aaa acc cta aga 3320
   Ile Glu Glu Glu Lys Ser Gln Ser Gln Val Ala Ile Lys Thr Leu Arg
   35 40 45 50
   agg tta ggt gct tca aat aac cct agt gga tta cca aga aaa aaa gat 3368
   Arg Leu Gly Ala Ser Asn Asn Pro Ser Gly Leu Pro Arg Lys Lys Asp
   55 60 65
   att gga gaa aaa agc aca ata ggg ttc cct aca atg aga caa gtt tca 3416
   Ile Gly Glu Lys Ser Thr Ile Gly Phe Pro Thr Met Arg Gln Val Ser
   70 75 80
   gtt tca gat aca tta cta aca aat gag ata ctt gta atg aga cga ata 3464
   Val Ser Asp Thr Leu Leu Thr Asn Glu Ile Leu Val Met Arg Arg Ile
   85 90 95
   gtc gaa aac gtt tcg cca cat cca aat gtg att gat ctt tat gat gta 3512
   Val Glu Asn Val Ser Pro His Pro Asn Val Ile Asp Leu Tyr Asp Val
   100 105 110
   tat gag gac aca aat ggt gtt cat ctt gtt ctt gag ctt tgt tcc ggt 3560
   Tyr Glu Asp Thr Asn Gly Val His Leu Val Leu Glu Leu Cys Ser Gly
   115 120 125 130
   ggt gaa ctt ttc gat agg att gtt gca caa gat aag tat agt gag act 3608
   Gly Glu Leu Phe Asp Arg Ile Val Ala Gln Asp Lys Tyr Ser Glu Thr
   135 140 145
   gaa gct gca act gtg gtt cat caa ata gct tca ggg tta gaa gct gtt 3656
   Glu Ala Ala Thr Val Val His Gln Ile Ala Ser Gly Leu Glu Ala Val
   150 155 160
   cat aga gct aat ata gtt cat aga gat ttg aaa cct gaa aat tgt ctt 3704
   His Arg Ala Asn Ile Val His Arg Asp Leu Lys Pro Glu Asn Cys Leu
   165 170 175
   ttt tta gat gtt agg aaa gat tct cct ctt aag att atg gat ttt ggg 3752
   Phe Leu Asp Val Arg Lys Asp Ser Pro Leu Lys Ile Met Asp Phe Gly
   180 185 190
   ttg agt tct gtt gaa gag ttt act gat cct gtt gtt ggt ttg ttt gga 3800
   Leu Ser Ser Val Glu Glu Phe Thr Asp Pro Val Val Gly Leu Phe Gly
   195                 200                 205                 210
   tct att gat tat gtt tca cct gag gct ctt tct caa gga aag att act      3848
   Ser Ile Asp Tyr Val Ser Pro Glu Ala Leu Ser Gln Gly Lys Ile Thr
                   215                 220                 225
   act aag agt gat atg tgg tct ctt ggg gtt att cta tat atc tta ctt      3896
   Thr Lys Ser Asp Met Trp Ser Leu Gly Val Ile Leu Tyr Ile Leu Leu
               230                 235                 240
   tca gg  gtatgttttt acttttcttt ctttcttata tttaaattgt attaggtttt      3951
   Ser Gly
   atgttttgat tctagcgatg atgagtttgt atgtatcatc gtggttttaa caaaactcca   4011
   ctttagatct tctacaaaat tacggtgcta ctgtgatttc gtcaaactca acatgtccaa   4071
   acatgtacta gtagtatgtt tagttttaaa tttggaattt acaaacctaa cctacaagaa   4131
   atagcgccga tttgggaaca cggtagctca catctcaccg cgtggtagct cgtctctcca   4191
   aattaatagt gcgtttggta ttatgtttgg aatttccaac ccaaacctac aagaaatagt   4251
   gtctgtttgg aaacatgtag cttacctctc acagcgaggt agctcattac ttcaagatga   4311
   acttcctcga agtgacatca taaaaaaaga aacccaaaca aacacatgct taagcttttg   4371
   ttagatacct tgtgatttcg ttaaactcaa cgtatccaaa catatactag tggtatgttt   4431
   agttttaaaa ttattttcat ataattgtta tgcag g tat cca cct ttc att gcc    4485
                                            Tyr Pro Pro Phe Ile Ala
                                            245                 250
   caa aat aat cgc caa aaa caa caa atg ata atg aat gtaagtacca          4531
   Gln Asn Asn Arg Gln Lys Gln Gln Met Ile Met Asn
                   255                 260
   tctcttacag aatatagaag ttctttccac aattactttg cttactaaaa caaaatggtt   4591
   tggaacag ggg aat ttt agt ttt tat gag aag act tgg aag gga att tca    4641
            Gly Asn Phe Ser Phe Tyr Glu Lys Thr Trp Lys Gly Ile Ser
                    265                 270                 275
   caa cca gca aag aat ttg att tca agt ctt tta acc gtt gat cct agc      4689
   Gln Pro Ala Lys Asn Leu Ile Ser Ser Leu Leu Thr Val Asp Pro Ser
               280                 285                 290
   aag aga cct agt gct ctt gag gtattgtaca ctactactat cttgaacata        4740
   Lys Arg Pro Ser Ala Leu Glu
           295
   ccaacttaac attaaatgtc cacataaata ttttttaatc gagtttgatg aaattagaat   4800
   aatgatgtag atattttatg acttaaagga ccaatttata acaaaaaaaa cttaaatgac   4860
   taacttgcta taatacaact taagaatcgt agatgatatt tgacctattt ttcatgacat   4920
   tatcttcaaa tgtaaagaag tttaaggtga ataaaaacca caacacacat gttgttgtac    4980
   tttgtgtttt ttggaacag ctt cta agt gat cca tgg gtc aaa ggt gag aaa     5032
                        Leu Leu Ser Asp Pro Trp Val Lys Gly Glu Lys
                        300                 305                 310
   gcc aaa gat gtt caa atg gac cct gag att gtc tca agg cta caa agc      5080
   Ala Lys Asp Val Gln Met Asp Pro Glu Ile Val Ser Arg Leu Gln Ser
                   315                 320                 325
   ttt aat gca aga cgt aaa ctt cgt gca gct gca att gct agt gtt tgg      5128
   Phe Asn Ala Arg Arg Lys Leu Arg Ala Ala Ala Ile Ala Ser Val Trp
               330                 335                 340
   agc tcc aca atc ttc ctt aga aca aaa aaa ttg aaa tca ttg gtt gga      5176
   Ser Ser Thr Ile Phe Leu Arg Thr Lys Lys Leu Lys Ser Leu Val Gly
           345                 350                 355
   tcc tat gat ctt aaa gaa gag gaa att gaa aat ctc agg atg cat ttc      5224
   Ser Tyr Asp Leu Lys Glu Glu Glu Ile Glu Asn Leu Arg Met His Phe
       360                 365                 370
   aag aag at  gtaagtaccc aaaagtcaaa aattatcata gtaacattta              5272
   Lys Lys Ile
   375
   actttagctc ttaaatttca caaggaacca aacattttaa gtaaataatc aaattagtta    5332
   agaatgacag ataattaaaa tattcaaaag tattcagaac tcctagctca actggcaaaa    5392
   atgtcgaaat tgttaggccg aatgccataa ccggggttct accaaaaaaa aaaatattca    5452
   aaagtaattt tcataatatt tacacaattc gagctaaggt tttaaaaaac agttcaaagt    5512
   cgcgtttgag atgtgacatc aatgtttttt tatgtctttg tagtaattac aattacattt    5572
   gctcgaagtg tattttgaca caaaaaattg ggactatgtg tacgatacac ttcggactaa    5632
   tgttttcttt ttgaatacaa agaaaatact aatagaaaat acaccatttc tgatttttca    5692
   atattaacaa gtgtgttgca gttgtaaact ttgatagttt tgattaaagt aatgtaatga    5752
   gttgttaaca g a tgt gca gat aga gac aat gca act ctg tca gag ttt       5800
                  Cys Ala Asp Arg Asp Asn Ala Thr Leu Ser Glu Phe
                          380                 385
   gag gag gtg tta aaa gca atg aat atg tta tca ttg atc cct ttt gct      5848
   Glu Glu Val Leu Lys Ala Met Asn Met Leu Ser Leu Ile Pro Phe Ala
   390                 395                 400                 405
   tct cgt ata ttt gat ttg ttt gac aac aac cgt gat gga aca gtt gac      5896
   Ser Arg Ile Phe Asp Leu Phe Asp Asn Asn Arg Asp Gly Thr Val Asp
                   410                 415                 420
   atg cgt gag ata ctt tgt gga ttt tcc agt ctc aag aat tcc aaa gga      5944
   Met Arg Glu Ile Leu Cys Gly Phe Ser Ser Leu Lys Asn Ser Lys Gly
               425                       430                       435
   gag gat gct ctt cgt ttg tgc ttc cag gtgaattata ctcaacaccc           5991
   Glu Asp Ala Leu Arg Leu Cys Phe Gln
           440                    445
   aaacaaatat taagttttac aaatatgaat atgtgaagca aatggattca aattatagat   6051
   atttgtgagt tgaaatttat gttgttagta tcagactaga tagttcgact agtttaaact   6111
   aaatgttaag gagtttagac attgaattta aacaacacta acgaaatagt aaatattaac   6171
   ctaaaattat gtatttaact tagttgatca acattggggc ctcataactt gttagacctg   6231
   tacattcaaa ccttagactt agtacaacat gatttatttt atttttctaa gtcttgataa   6291
   aacgttcact atatatttag gcttaatttt ttttgtcaag ttttacttac cttcaagctg   6351
   aattacggat tattaaagtc tcgttcacct aagaatcgaa gagttaacac caaaataaaa   6411
   taaaaattac ttttgacctt taaatatgtt ggactgaatc aataaattag ttttttttta   6471
   gaggttgaac caataaatta gtcttttttt taaggaataa attaatgtct ctaaagaaat   6531
   agcttaaaaa ttaatgttat gaacatacac tctataacta gttttcaaca tgttaaccac   6591
   atttgcctct ttatttttat tttgttggca aatag atg tat gat aca gat aga      6644
                                          Met Tyr Asp Thr Asp Arg
                                                          450
   tca ggc tgc atc agc aaa gag gaa gta gca tcc atg ctc agg             6686
   Ser Gly Cys Ile Ser Lys Glu Glu Val Ala Ser Met Leu Arg
           455                    460                    465
   gtaatgcaca cacatctcaa ttaattagct tcatcttttc actttcatta tattaacaca   6746
   catttataca ttaattaaac ag gct ttg cca tat gat tgt ctt cca act gat    6798
                            Ala Leu Pro Tyr Asp Cys Leu Pro Thr Asp
                                            470                    475
   atc act gaa cct gga aaa ttg gat gag att ttt gac tta atg gat gct     6846
   Ile Thr Glu Pro Gly Lys Leu Asp Glu Ile Phe Asp Leu Met Asp Ala
               480                    485                    490
   aat aat gat gga aaa gtt aca ttt gat gaa ttc aaa gct gct atg caa     6894
   Asn Asn Asp Gly Lys Val Thr Phe Asp Glu Phe Lys Ala Ala Met Gln
           495                    500                    505
   aga gat agc tct ctt caa gat gta gtt ctc tct tct att cgt cca taa     6942
   Arg Asp Ser Ser Leu Gln Asp Val Val Leu Ser Ser Ile Arg Pro
       510                    515                    520
   tagttttatg ttcatgatgt tatggtattt taatacttga gtacttttat gctcctttga   7002
   ggactttttt atgaagtttt cgtagtaaaa catagtgttc aggcccgacc ctgatatttt   7062
   tggtgccctt atcgagtaaa ataatcatat cattcaacaa cttatcgagt attccaccat   7122
   caatgtgaag tgttggtcaa ttcaacaact ctcgtgaatc gtttgctgtc tcttctaggg   7182
   tttcactgtt cgccgtctac ccctgtgagt gtgatcgtcg tttgtttagg ttctcataat   7242
   ttgtatgttt tggatcttgc ctaatctatg tatcatgtat gattatggtg atcaagtatg   7302
   catgcgtatg tggtaattgg attagatttc aattgatggg tcttacatta gatttgttgc   7362
   atagataata gtttttttat ctctcccaca aaaatcctca agcgtgtctc tatttcaagc   7422
   tactcgatct ggttatatgt gttggtatga tctggattcc atggacggtt tcaagacccg   7482
   gcgagcccgg gtagtcgccc aagctctggc ccaaattttt tgcaatttct tttgtaaatc   7542
   tccttgaatt tcttatataa acccctataa attgggtaat ttctctatct agtattttat   7602
   tatttgcttg caatttttta tataaacccc tataaatgtg ttgaattttt tttttgccaa   7662
   ggctttataa taatcctgga tccgcatact atttatcatc ccctagtaga gagtcgaatt   7722
   aagcttggca tatggttacc atgtgttctt atcaaacatt agtctataaa aaatagtatt   7782
   ataagttgga attaccaagg cttaggaaac ctgagtgtat agcttcaaaa ttgaaataca   7842
   tagtccgtta ctataagcca aatgctttat ttctaaatga actttagtcc actccaataa   7902
   aactgaagaa ttacgttgtt tgcttgattt tgatagttgt ttagcaatta gcagtcttcg   7962
   tctaaatggt ggcttagcgc tatcttggag caactctttt aattgtattg tgttaaatta   8022
   tatgcaaatc atattaatgt agaagttcat gatcctcgac aaggtaaatg acaattcaca   8082
   agtttttaca acttcttgga agagggagaa ggtgtgcctc atgggatttc ttgcataata   8142
   tgtcacaaag tatttcgctc ccatggtgta ttatgggtga ctttattgat atcatgggtg   8202
   tccgtgagaa acggggtggc tatgaacgtt ctatgtggtt gataagcggt ttcaggcaac   8262
   catggctcag tagtggatcc ctcccatcta actagccttt ggaaacgaat gacgcatctg   8322
   atgatacaag aagacaagta gtggagacag cgcactaaga aacattggta tagagacgat   8382
   gatcttaata caaaattttt acatgcctct gccacttctc ggaaaaaggt aatccgcatt   8442
   ttttccttag aaaacaacgt tggtgaggga atttcatatg ataatggtat gtgtcaggtt   8502
   gcaaaggggt attttgaaga attgttttta ggaagaaaaa tagcattaga gcttcggttg   8562
   ttggtgctat tttagaggta gttatagatg aggatagcaa tttccttact actccttttc   8622
   atacacaaga gttcaaggaa cccttgtttt ccatgcaccg gacaagtgtc caggaccgaa   8682
   tggtttcaat tatgtttttt tttttaaaca ttttttgtct gtttgtagcc ctgatacatt   8742
   tcataaatgt tacacatggt tgagtaataa ccaaatccct tcctctctaa actttgataa   8802
   cattgccttg attcctcggg ggaatgaaca taagacaatg aaggattggc gaccaattgc   8862
   attgtgtata ttctttataa attacatgta aaagttcttg ctaaccgact taagcatata   8922
   ttacacaaat gcatctgtga caataagtca ccttttgttt gagagtcttt ttgtaacaat   8982
   ttttgttttt tctcatttga tttaactata ctcttttttc tagttctttt ggcacttctg   9042
   gtgctataac ttcatgtgtg ttttaatata tttcatttta ccttcttcaa aaataaatat   9102
   tttaaaaaat atatataaat tgtttttttt aattattttc ctgaaaacga gaattttacg   9162
   tgtaacaaat tttaattact gaaaagataa aaatattttg atttctaatt aaattgaaat   9222
   aaaaaaaaaa atcaattttg aatgttatct aaatatgaat catacacaat tgattcttca   9282
   tgattcggat gtattaaata tgactaattg agtcatgcat tttttgagtc atacatgttt   9342
   ctcgttaaaa acacatctaa ccagtggcgg agccacgtat tggtcagctg gtgcagctgc   9402
   atcccctcaa agttttaaat ttagaccaat atttcttatt ttattcattt tgaacctcct   9462
   caaattataa ctttgcacca gcttaaatct tgtcactgca caatggtaat aataatagtg   9522
   acgtatatgc agctaataat aataggtcaa aacaacgtag ccattgggct ggaccctact    9582
   aaatgtagtg atatatgcag ctcataataa taggccaata tatatgttac acttaatact    9642
   tgaaaaaaaa ctatgggaca ctcaaaaatt taatttgtat caatatattc ggtctttaaa    9702
   ttaatagtta aatgcgttat attttaaaaa aaattacaat aactaaattt attatccaaa    9762
   gtatcgacga aataaaattt attgattgtt ttattacgat aaaatataaa aaatttgata    9822
   tgattcttat aaacaatgag atgatatttt tttttcttat gaaatgacat tttataaaat    9882
   accaatttga aaaataactc tttatttcat aaaaataatt ggttatttta aaaaacacgt    9942
   gtaccggtac acttaaaatg taacttgaca tcaatttgga tcaaatttaa gagactaaac   10002
   tcttacaatg tgatagacta atgtttaaat ctgacttaaa gagatacttg tgttttgtac   10062
   ttgacatatt tcgaaactca aataagatta tcctcaacaa gaagaactta taaaaagaaa   10122
   tattaatttg aggataaaaa tgtaaagagg ataaaaatgt gaggtaaata atatttttat   10182
   taaaatttag ttattctagt atttatgaat aaaccataat tgtatcttta tcttcataca   10242
   tacatatata attataataa tatgtacaaa aaaaataaca ataatgatga taaatttttt   10302
   taaaaaaatt gcacctcctg acccgaggtc ctggctacgc cactgcatct aactacttac   10362
   aagacaccac caatatgtct tatatcttat aatatacatg tttttctatg cttcatgtat   10422
   taaatatgat ttttgaagtc agggttcggc cctttaaatt tggatgtctt ggacgaatca   10482
   agagagtggt gtccttataa aaaaaatttc acaaaaattg ataagattaa gagaaatgat   10542
   ctaaaagact attaaacatt tcatttgaca ttgtacaaga aaaacacaag tatgaatcat   10602
   tgaagtgtga accaattagc tattattcat cttccttttc ctcttctttg atttgttatt   10662
   gtttcctata aaaaaaattg accaagcatt taaaactatt taaatatttt cttcgttgaa   10722
   ttttttgttg caaaatcatc aataattcgt tcaaaatcaa ggttcttcac aaaaaatcca   10782
   tctgtttatt attcactcca tatcattatt tcaatttaag taccccaatt aaactattga   10842
   ctatttactt gctttccaca aattactcat cagttcattt gctaattgat tttttggtac   10902
   atgaattcaa atattaaaaa agtgtaacaa acacaaattt tttgattacc atacaaaata   10962
   tatcatgtaa caaacacgat atgagtatat gtagattttt cctttatcct taataaaagt   11022
   gtaacaaact agatcaaata tatgttctta taaatataat caataagaaa aggaaaatta   11082
   tcatcgatac atcgagataa aatattttat atacaggcaa accaaaatag gtcgagttga   11142
   taaataattt tgttatatgc tttgacaata atttgtagat acatcgacgt acagataa     11200
<210> 2
   <211> 523
   <212> PRT
   <213> Medicago truncatula
<400> 2
   Met Gly Tyr Gly Thr Arg Lys Leu Ser Asp Glu Tyr Glu Val Ser Glu
   1               5                   10                  15
   Ile Leu Gly Arg Gly Gly Phe Ser Val Val Arg Lys Gly Thr Lys Lys
   20 25 30
   Ser Ser Ile Glu Glu Glu Lys Ser Gln Ser Gln Val Ala Ile Lys Thr
   35 40 45
   Leu Arg Arg Leu Gly Ala Ser Asn Asn Pro Ser Gly Leu Pro Arg Lys
   50 55 60
   Lys Asp Ile Gly Glu Lys Ser Thr Ile Gly Phe Pro Thr Met Arg Gln
   65 70 75 80
   Val Ser Val Ser Asp Thr Leu Leu Thr Asn Glu Ile Leu Val Met Arg
   85 90 95
   Arg Ile Val Glu Asn Val Ser Pro His Pro Asn Val Ile Asp Leu Tyr
   100 105 110
   Asp Val Tyr Glu Asp Thr Asn Gly Val His Leu Val Leu Glu Leu Cys
   115 120 125
   Ser Gly Gly Glu Leu Phe Asp Arg Ile Val Ala Gln Asp Lys Tyr Ser
   130 135 140
   Glu Thr Glu Ala Ala Thr Val Val His Gln Ile Ala Ser Gly Leu Glu
   145 150 155 160
   Ala Val His Arg Ala Asn Ile Val His Arg Asp Leu Lys Pro Glu Asn
   165 170 175
   Cys Leu Phe Leu Asp Val Arg Lys Asp Ser Pro Leu Lys Ile Met Asp
   180 185 190
   Phe Gly Leu Ser Ser Val Glu Glu Phe Thr Asp Pro Val Val Gly Leu
   195 200 205
   Phe Gly Ser Ile Asp Tyr Val Ser Pro Glu Ala Leu Ser Gln Gly Lys
   210 215 220
   Ile Thr Thr Lys Ser Asp Met Trp Ser Leu Gly Val Ile Leu Tyr Ile
   225 230 235 240
   Leu Leu Ser Gly Tyr Pro Pro Phe Ile Ala Gln Asn Asn Arg Gln Lys
   245 250 255
   Gln Gln Met Ile Met Asn Gly Asn Phe Ser Phe Tyr Glu Lys Thr Trp
   260 265 270
   Lys Gly Ile Ser Gln Pro Ala Lys Asn Leu Ile Ser Ser Leu Leu Thr
   275 280 285
   Val Asp Pro Ser Lys Arg Pro Ser Ala Leu Glu Leu Leu Ser Asp Pro
   290 295 300
   Trp Val Lys Gly Glu Lys Ala Lys Asp Val Gln Met Asp Pro Glu Ile
   305 310 315 320
   Val Ser Arg Leu Gln Ser Phe Asn Ala Arg Arg Lys Leu Arg Ala Ala
   325 330 335
   Ala Ile Ala Ser Val Trp Ser Ser Thr Ile Phe Leu Arg Thr Lys Lys
   340 345 350
   Leu Lys Ser Leu Val Gly Ser Tyr Asp Leu Lys Glu Glu Glu Ile Glu
   355 360 365
   Asn Leu Arg Met His Phe Lys Lys Ile Cys Ala Asp Arg Asp Asn Ala
   370 375 380
   Thr Leu Ser Glu Phe Glu Glu Val Leu Lys Ala Met Asn Met Leu Ser
   385 390 395 400
   Leu Ile Pro Phe Ala Ser Arg Ile Phe Asp Leu Phe Asp Asn Asn Arg
   405 410 415
   Asp Gly Thr Val Asp Met Arg Glu Ile Leu Cys Gly Phe Ser Ser Leu
   420 425 430
   Lys Asn Ser Lys Gly Glu Asp Ala Leu Arg Leu Cys Phe Gln Met Tyr
   435 440 445
   Asp Thr Asp Arg Ser Gly Cys Ile Ser Lys Glu Glu Val Ala Ser Met
   450 455 460
   Leu Arg Ala Leu Pro Tyr Asp Cys Leu Pro Thr Asp Ile Thr Glu Pro
   465 470 475 480
   Gly Lys Leu Asp Glu Ile Phe Asp Leu Met Asp Ala Asn Asn Asp Gly
   485 490 495
   Lys Val Thr Phe Asp Glu Phe Lys Ala Ala Met Gln Arg Asp Ser Ser
   500 505 510
   Leu Gln Asp Val Val Leu Ser Ser Ile Arg Pro
   515 520
<210> 3
   <211> 2586
   <212> DNA
   <213> Pisum sativum
<220>
   <221> exon
   <222> (294) .. (1048)
   <223>
<220>
   <221> exon
   <222> (1219)..(1273)
   <223>
<220>
   <221> exon
   <222> (1349)..(1459)
   <223>
<220>
   <221> exon
   <222> (1541)..(1773)
   <223>
<220>
   <221> exon
   <222> (1978)..(2192)
   <223>
<220>
   <221> exon
   <222> (2284)..(2330)
   <223>
<220>
   <221> exon
   <222> (2416) .. (2586)
   <223>
<400> 3
   cctcttcttt gtccattcta aaacacctct taccaaccta cttaatattc ccttctatct     60
   ctcttaattt aattcatatt ttatttcata aacaattcaa ataactctta tagtattatt    120
   ataacctata atgtctattt caaccatttc aataacgcgt tttatatctt cattattgaa    180
   catattatca ttattacata tcctcttgtt cttttcctct tcatacattt tcttactctt    240
   caaaatatat tccatttttt caaactcttt tgtttttgtt aattatcact acc atg       296
                                                               Met
                                                               1
   gaa tat ggt aca aga aaa ctc tca gat gtg tat gaa gtt tca gag att      344
   Glu Tyr Gly Thr Arg Lys Leu Ser Asp Val Tyr Glu Val Ser Glu Ile
               5                   10                  15
   cta gga aga ggt gga ttt tct gtt gtt aga aaa ggt aca aga aaa tca      392
   Leu Gly Arg Gly Gly Phe Ser Val Val Arg Lys Gly Thr Arg Lys Ser
           20                  25                  30
   aac aat gat gat gaa aaa tca caa tca caa tca aaa tca caa tca caa      440
   Asn Asn Asp Asp Glu Lys Ser Gln Ser Gln Ser Lys Ser Gln Ser Gln
       35                  40                  45
   tca caa gta gca atc aaa aca ctt aga aga tta ggt act tca aat aat      488
   Ser Gln Val Ala Ile Lys Thr Leu Arg Arg Leu Gly Thr Ser Asn Asn
   50                  55                  60                  65
   tta cca agg aaa aaa gat ggt ggt gaa aat agc aca gaa act atg atg      536
   Leu Pro Arg Lys Lys Asp Gly Gly Glu Asn Ser Thr Glu Thr Met Met
                   70                  75                  80
   aag ttc cct aca atg aga caa gtt tct gtc tca gat gca tta cta aca      584
   Lys Phe Pro Thr Met Arg Gln Val Ser Val Ser Asp Ala Leu Leu Thr
               85                  90                  95
   aat gag att ctt gta atg aga aga ata gtc gaa aac gtt tcg ccg cat      632
   Asn Glu Ile Leu Val Met Arg Arg Ile Val Glu Asn Val Ser Pro His
           100                 105                 110
   ccg aac gtt atc gat ctt tac gat gtt tat gaa gac aca aat ggt gtt      680
   Pro Asn Val Ile Asp Leu Tyr Asp Val Tyr Glu Asp Thr Asn Gly Val
       115                 120                 125
   cat ctt gtg ctt gag ctt tgt tct ggt ggc gaa ctt ttc gat agg att      728
   His Leu Val Leu Glu Leu Cys Ser Gly Gly Glu Leu Phe Asp Arg Ile
   130                 135                 140                 145
   gtt gca caa gat aag tat agt gag act gaa gct tca act gtg gtt cat      776
   Val Ala Gln Asp Lys Tyr Ser Glu Thr Glu Ala Ser Thr Val Val His
                   150                 155                 160
   cag att gtt gca ggg tta gaa gct att cat aga gct aac att att cat      824
   Gln Ile Val Ala Gly Leu Glu Ala Ile His Arg Ala Asn Ile Ile His
               165                 170                 175
   agg gat ttg aag cct gaa aat tgt ctt ttt tta gat gtt ggg aaa gat      872
   Arg Asp Leu Lys Pro Glu Asn Cys Leu Phe Leu Asp Val Gly Lys Asp
           180                 185                 190
   tct agt ctt aag att atg gat ttt ggg ttg agt tct gtt gaa gag ttt      920
   Ser Ser Leu Lys Ile Met Asp Phe Gly Leu Ser Ser Val Glu Glu Phe
       195                 200                 205
   act gat cct gtt gtt ggt ttg ttt gga tct att gat tat gtt tct cct      968
   Thr Asp Pro Val Val Gly Leu Phe Gly Ser Ile Asp Tyr Val Ser Pro
   210                 215                 220                 225
   gag gct ctt tct caa gga aag att act act aag agt gat atg tgg tct     1016
   Glu Ala Leu Ser Gln Gly Lys Ile Thr Thr Lys Ser Asp Met Trp Ser
                   230                 235                 240
   ctt gga gtc att cta tat atc tta ctt tcc gg gtatgttttt ttcttttgag   1068
   Leu Gly Val Ile Leu Tyr Ile Leu Leu Ser Gly
               245                 250
   tatctgtacg agtaacagga gaaactaatt ctttaaacga gagaggacta caatagacga   1128
   caaagtattt tctcaagaga tttaacactg ttgtattaag ttggaagaaa tctcttacca   1188
   tctcatctaa ttgctttgat tgtttttcag g tat cca cct ttc att gct caa     1240
                                      Tyr Pro Pro Phe Ile Ala Gln
                                                  255
   aat aat cgc caa aag caa caa atg ata ttg aat gtaagtaaca tcttagacat   1293
   Asn Asn Arg Gln Lys Gln Gln Met Ile Leu Asn
   260                 265                 270
   gataaattca tttcataatg tttttttgtt tactaaataa aatgttttgg aacag gga    1351
                                                                Gly
   aat ttt agt ttt tat gag aag act tgg aag gga att tca caa tca gca     1399
   Asn Phe Ser Phe Tyr Glu Lys Thr Trp Lys Gly Ile Ser Gln Ser Ala
               275                 280                 285
   aag aat ttg att tca agt ctt cta act gtt gat cct gct aaa aga cct     1447
   Lys Asn Leu Ile Ser Ser Leu Leu Thr Val Asp Pro Ala Lys Arg Pro
           290                 295                 300
   agt gca caa gag gtactctaat tctacactct taataactta agtaattgaa         1499
   Ser Ala Gln Glu
       305
   aatattgaag tgttttcaat ttttttttgt tattggaaca g ctt tta agt gat cca   1555
                                                 Leu Leu Ser Asp Pro
                                                             310
   tgg gtt aaa ggt gag aag gcc aaa gat gat caa atg gac cct gaa att     1603
   Trp Val Lys Gly Glu Lys Ala Lys Asp Asp Gln Met Asp Pro Glu Ile
           315                 320                 325
   gtg tca agg cta caa aga ttc aat gca aga cgc aaa ctt cgc gca gct     1651
   Val Ser Arg Leu Gln Arg Phe Asn Ala Arg Arg Lys Leu Arg Ala Ala
       330                 335                 340
   gca att gct agt gtt tgg agc tcc aca att ttt cta aga act aaa aaa     1699
   Ala Ile Ala Ser Val Trp Ser Ser Thr Ile Phe Leu Arg Thr Lys Lys
   345                 350                 355                 360
   ttg aaa tcc ttg gtt gga tcc tat gat ctc aaa gaa gat gaa att gaa     1747
   Leu Lys Ser Leu Val Gly Ser Tyr Asp Leu Lys Glu Asp Glu Ile Glu
                   365                 370                 375
   aat ctt agg atg cac ttc aag aaa at  gtaagtacag tattccaaat           1793
   Asn Leu Arg Met His Phe Lys Lys Ile
               380
   cgcatttgta atgacatgag cgttttttaa atgtctgtgt cattgtcaat gtaatttcta   1853
   ttaagtatat ttaacatcaa attttcaata ataagaaaat cgtgaggcgg attgagacta   1913
   ctatttaaaa tcttggttcc agctgtgatt ttgatttaac taatggtgct atggttgtta   1973
   acag a tgt gca gat aga gac aat gcg act ctt tgt gag ttt gaa gag      2020
          Cys Ala Asp Arg Asp Asn Ala Thr Leu Cys Glu Phe Glu Glu
                         390                 395
   gtg tta aaa gca atg aaa atg cca tca ttg atc cct ttc gca gct cgt     2068
   Val Leu Lys Ala Met Lys Met Pro Ser Leu Ile Pro Phe Ala Ala Arg
   400                 405                 410                 415
   ata ttt gat tta ttt gac aac aac cgc gat gga acg gtt gac atg aga     2116
   Ile Phe Asp Leu Phe Asp Asn Asn Arg Asp Gly Thr Val Asp Met Arg
                   420                 425                 430
   gag att ctt tgt ggc ttt tcg agt ctc aag aac tcc aaa gga gag gat     2164
   Glu Ile Leu Cys Gly Phe Ser Ser Leu Lys Asn Ser Lys Gly Glu Asp
               435                 440                 445
   gct ctc cgt ttg tgt ttt cag gca agg g gtgtgcaaaa atatgattaa         2212
   Ala Leu Arg Leu Cys Phe Gln Ala Arg
           450                 455
   ttgaaacata tttccaacat gtgaacgcat ttctgatttt atttttgttg gcaattagat   2272
   gtatgacaca g at  aga tca gga tgc atc acc aag gaa gaa gta gca tct    2321
                Asp Arg Ser Gly Cys Ile Thr Lys Glu Glu Val Ala Ser
                            460                 465
   atg cta aga gtaatgttca ttcctctcaa tgaattagct ttatattttc            2370
   Met Leu Arg
   470
   actgtcatct tttaacacaa gtgtatatat atatttccta aacag gct ttg cca tat  2427
                                                     Ala Leu Pro Tyr
   475
   gat tgt ctt cca act gat atc act gaa cct gga aaa ttg gat gag att 2475
   Asp Cys Leu Pro Thr Asp Ile Thr Glu Pro Gly Lys Leu Asp Glu Ile
   480 485 490
   ttt gac tta atg gat gcc aat agt gat gga aaa gtt aca ttt gat gaa 2523
   Phe Asp Leu Met Asp Ala Asn Ser Asp Gly Lys Val Thr Phe Asp Glu
   495 500 505
   ttc aaa gct gca atg caa aga gat agc tct ctt caa gat gta gtt ctc 2571
   Phe Lys Ala Ala Met Gln Arg Asp Ser Ser Leu Gln Asp Val Val Leu
   510 515 520
   tct tct att cgt cca 2586
   Ser Ser Ile Arg Pro
   525
<210> 4
   <211> 529
   <212> PRT
   <213> Pisum sativum
<400> 4
   Met Glu Tyr Gly Thr Arg Lys Leu Ser Asp Val Tyr Glu Val Ser Glu
   1 5 10 15
   Ile Leu Gly Arg Gly Gly Phe Ser Val Val Arg Lys Gly Thr Arg Lys
   20 25 30
   Ser Asn Asn Asp Asp Glu Lys Ser Gln Ser Gln Ser Lys Ser Gln Ser
   35 40 45
   Gln Ser Gln Val Ala Ile Lys Thr Leu Arg Arg Leu Gly Thr Ser Asn
   50 55 60
   Asn Leu Pro Arg Lys Lys Asp Gly Gly Glu Asn Ser Thr Glu Thr Met
   65 70 75 80
   Met Lys Phe Pro Thr Met Arg Gln Val Ser Val Ser Asp Ala Leu Leu
   85 90 95
   Thr Asn Glu Ile Leu Val Met Arg Arg Ile Val Glu Asn Val Ser Pro
   100 105 110
   His Pro Asn Val Ile Asp Leu Tyr Asp Val Tyr Glu Asp Thr Asn Gly
   115 120 125
   Val His Leu Val Leu Glu Leu Cys Ser Gly Gly Glu Leu Phe Asp Arg
   130 135 140
   Ile Val Ala Gln Asp Lys Tyr Ser Glu Thr Glu Ala Ser Thr Val Val
   145 150 155 160
   His Gln Ile Val Ala Gly Leu Glu Ala Ile His Arg Ala Asn Ile Ile
   165 170 175
   His Arg Asp Leu Lys Pro Glu Asn Cys Leu Phe Leu Asp Val Gly Lys
   180 185 190
   Asp Ser Ser Leu Lys Ile Met Asp Phe Gly Leu Ser Ser Val Glu Glu
   195 200 205
   Phe Thr Asp Pro Val Val Gly Leu Phe Gly Ser Ile Asp Tyr Val Ser
   210 215 220
   Pro Glu Ala Leu Ser Gln Gly Lys Ile Thr Thr Lys Ser Asp Met Trp
   225 230 235 240
   Ser Leu Gly Val Ile Leu Tyr Ile Leu Leu Ser Gly Tyr Pro Pro Phe
   245 250 255
   Ile Ala Gln Asn Asn Arg Gln Lys Gln Gln Met Ile Leu Asn Gly Asn
   260 265 270
   Phe Ser Phe Tyr Glu Lys Thr Trp Lys Gly Ile Ser Gln Ser Ala Lys
   275 280 285
   Asn Leu Ile Ser Ser Leu Leu Thr Val Asp Pro Ala Lys Arg Pro Ser
   290 295 300
   Ala Gln Glu Leu Leu Ser Asp Pro Trp Val Lys Gly Glu Lys Ala Lys
   305 310 315 320
   Asp Asp Gln Met Asp Pro Glu Ile Val Ser Arg Leu Gln Arg Phe Asn
   325 330 335
   Ala Arg Arg Lys Leu Arg Ala Ala Ala Ile Ala Ser Val Trp Ser Ser
   340 345 350
   Thr Ile Phe Leu Arg Thr Lys Lys Leu Lys Ser Leu Val Gly Ser Tyr
   355 360 365
   Asp Leu Lys Glu Asp Glu Ile Glu Asn Leu Arg Met His Phe Lys Lys
   370 375 380
   Ile Cys Ala Asp Arg Asp Asn Ala Thr Leu Cys Glu Phe Glu Glu Val
   385 390 395 400
   Leu Lys Ala Met Lys Met Pro Ser Leu Ile Pro Phe Ala Ala Arg Ile
   405 410 415
   Phe Asp Leu Phe Asp Asn Asn Arg Asp Gly Thr Val Asp Met Arg Glu
   420 425 430
   Ile Leu Cys Gly Phe Ser Ser Leu Lys Asn Ser Lys Gly Glu Asp Ala
   435 440 445
   Leu Arg Leu Cys Phe Gln Ala Arg Asp Arg Ser Gly Cys Ile Thr Lys
   450 455 460
   Glu Glu Val Ala Ser Met Leu Arg Ala Leu Pro Tyr Asp Cys Leu Pro
   465 470 475 480
   Thr Asp Ile Thr Glu Pro Gly Lys Leu Asp Glu Ile Phe Asp Leu Met
   485 490 495
   Asp Ala Asn Ser Asp Gly Lys Val Thr Phe Asp Glu Phe Lys Ala Ala
   500 505 510
   Met Gln Arg Asp Ser Ser Leu Gln Asp Val Val Leu Ser Ser Ile Arg
   515 520 525
   Pro
<210> 5
   <211> 20
   <212> DNA
   <213> Artificial sequence
<220>
   <223> PCR primer
<400> 5
   ggccaaatgt tgaacagtga 20
<210> 6
   <211> 22
   <212> DNA
   <213> Artificial sequence
<220>
   <223> PCR primer
<400> 6
   gggttttgat tgctacttgt ga 22
<210> 7
   <211> 20
   <212> DNA
   <213> Artificial sequence
<220>
   <223> PCR primer
   <400> 7
   tcaccatggg atatggaaca 20
<210> 8
   <211> 20
   <212> DNA
   <213> Artificial sequence
<220>
   <223> PCR primer
<400> 8
   ccacagttgc agcttcagtc 20
<210> 9
   <211> 20
   <212> DNA
   <213> Artificial sequence
<220>
   <223> PCR primer
<400> 9
   ctttgttccg gtggtgaact 20
<210> 10
   <211> 21
   <212> DNA
   <213> Artificial sequence
<220>
   <223> PCR primer
<400> 10
   tggacatgtt gagtttgacg a 21
<210> 11
   <211> 22
   <212> DNA
   <213> Artificial sequence
<220>
   <223> PCR primer
<400> 11
   tcgtggtttt aacaaaactc ca 22
<210> 12
   <211> 20
   <212> DNA
   <213> Artificial sequence
<220>
   <223> PCR primer
<400> 12
   attttgggca atgaaaggtg 20
<210> 13
   <211> 24
   <212> DNA
   <213> Artificial sequence
<220>
   <223> PCR primer
<400> 13
   tttcgttaaa ctcaacgtat ccaa 24
<210> 14
   <211> 25
   <212> DNA
   <213> Artificial sequence
<220>
   <223> PCR primer
<400> 14
   catttgaaga taatgtcatg aaaaa 25
<210> 15
   <211> 26
   <212> DNA
   <213> Artificial sequence
<220>
   <223> PCR primer
<400> 15
   tgacttaaag gaccaattta taacaa 26
<210> 16
   <211> 20
   <212> DNA
   <213> Artificial sequence
<220>
   <223> PCR primer
<400> 16
   tcgacatttt tgccagttga 20
<210> 17
   <211> 22
   <212> DNA
   <213> Artificial sequence
<220>
   <223> PCR primer
<400> 17
   aaatttcaca aggaaccaaa ca 22
<210> 18
   <211> 23
   <212> DNA
   <213> Artificial sequence
<220>
   <223> PCR primer
<400> 18
   gaagcaaaag ggatcaatga taa 23
<210> 19
   <211> 22
   <212> DNA
   <213> Artificial sequence
<220>
   <223> PCR primer
<400> 19
   tgtgcagata gagacaatgc aa 22
<210> 20
   <211> 20
   <212> DNA
   <213> Artificial sequence
<220>
   <223> PCR primer
<400> 20
   caagttatga ggccccaatg 20
<210> 21
   <211> 20
   <212> DNA
   <213> Artificial sequence
<220>
   <223> PCR primer
<400> 21
   caacattggg gcctcataac 20
<210> 22
   <211> 21
   <212> DNA
   <213> Artificial sequence
<220>
   <223> PCR primer
<400> 22
   caatcatatg gcaaagcctg t 21
<210> 23
   <211> 20
   <212> DNA
   <213> Artificial sequence
<220>
   <223> PCR primer
<400> 23
   atccatgctc agggtaatgc 20
<210> 24
   <211> 20
   <212> DNA
   <213> Artificial sequence
<220>
   <223> PCR primer
<400> 24
   acgacgatca cactcacagg 20
<210> 25
   <211> 24
   <212> DNA
   <213> Artificial sequence
<220>
   <223> PCR primer
<400> 25
   tcattgatcc cttttgcttc tcgt 24
<210> 26
   <211> 25
   <212> DNA
   <213> Artificial sequence
<220>
   <223> PCR primer
<400> 26
   gatgctactt cctctttgct gatgc 25
<210> 27
   <211> 26
   <212> DNA
   <213> Artificial sequence
<220>
   <223> PCR primer
<400> 27
   tggcatcact cagtaccttt caacag 26
<210> 28
   <211> 28
   <212> DNA
   <213> Artificial sequence
<220>
   <223> PCR primer
<400> 28
   acccaaagca tcaaataata agtcaacc 28
<210> 29
   <211> 20
   <212> DNA
   <213> Artificial sequence
<220>
   <223> PCR primer
<400> 29
   ggtggcgaac ttttcgatag 20
<210> 30
   <211> 22
   <212> DNA
   <213> Artificial sequence
<220>
   <223> PCR primer
<400> 30
   acctgaaaaa caatcaaagc aa 22
<210> 31
   <211> 22
   <212> DNA
   <213> Artificial sequence
<220>
   <223> PCR primer
<400> 31
   gcagttgact ctctctttgt cc 22
<210> 32
   <211> 21
   <212> DNA
   <213> Artificial sequence
<220>
   <223> PCR primer
<400> 32
   tctgtgctat tttcaccacc a 21
<210> 33
   <211> 25
   <212> DNA
   <213> Artificial sequence
<220>
   <223> PCR primer
<400> 33
   aacgagagag gactacaata gacga 25
<210> 34
   <211> 20'
   <212> DNA
   <213> Artificial sequence
<220>
   <223> PCR primer
<400> 34
   tcaaaatcac agctggaacc 20
<210> 35
   <211> 22
   <212> DNA
   <213> Artificial sequence
<220>
   <223> PCR primer
<400> 35
   ggaagaggtg gattttctgt tg 22
<210> 36
   <211> 21
   <212> DNA
   <213> Artificial sequence
<220>
   <223> PCR primer
<400> 36
   tcaggcttca aatccctatg a 21
<210> 37
   <211> 22
   <212> DNA
   <213> Artificial sequence
<220>
   <223> PCR primer
<400> 37
   aaaattgaaa tccttggttg ga 22
<210> 38
   <211> 20
   <212> DNA
   <213> Artificial sequence
<220>
   <223> PCR primer
<400> 38
   aacacaaacg gagagcatcc 20
<210> 39
   <211> 20
   <212> DNA
   <213> Artificial sequence
<220>
   <223> PCR primer
<400> 39
   agaaaatcgt gaggcggatt 20
<210> 40
   <211> 20
   <212> DNA
   <213> Artificial sequence
<220>
   <223> PCR primer
<400> 40
   ttggtgatgc atcctgatct 20
<210> 41
   <211> 20
   <212> DNA
   <213> Artificial sequence
<220>
   <223> PCR primer
<400> 41
   ttgtggcttt tcgagtctca 20
<210> 42
   <211> 24
   <212> DNA
   <213> Artificial sequence
<220>
   <223> PCR primer
<400> 42
   gcagctttga attcatcaaa tgta 24
<210> 43
   <211> 25
   <212> DNA
   <213> Artificial sequence
<220>
   <223> PCR primer
<400> 43
   tggacgaata gaagagagaa ctaca 25

## Description

The invention relates to polypeptides and polynucleotides involved in nodulation and mycorrhization in plants.

A wide range of terrestrial plants are able to associate, through their root system, with microbial symbionts.

The best known of these endosymbiotic systems are those occurring between soil bacteria collectively named rhizobia and roots of many legume (family Leguminosae) species. The legume-rhizobia symbiosis fixes as much nitrogen worldwide as the chemical fertilizer industry due to the ability of rhizobial bacteria to induce the morphogenesis of a new plant organ, the legume root nodule, in which they fix nitrogen.

Another important type of symbiotic relationships between plants and microorganisms is the arbuscular mycorrhizae (AM) symbiosis, that occurs not only in legumes but in most land plants (a notable exception is the Brassicaceae family which includes *Arabidopsis thaliana*) and involves fungi of the order *Glomales* (GIANINAZZI-PEARSON, Plant Cell, vol. 8, p.1871, 1996; HARRISON, Annu. Rev. Plant Physiol. Plant Mol. Biol., vol. 50, p.361, 1999 ; BRUNDRETT, New phytologist, vol. 154, p.275, 2002).

Although the legume-rhizobia symbiosis and the AM symbiosis may at first sight appear not to have much in common, both the fungal and bacterial partners possess the highly restricted ability to trigger a plant host genetic program permitting controlled and localized infection.

Studies on the legume-rhizobia symbiosis have led to the identification of the structure and importance of rhizobial signals, the lipochito-oligosaccharidic Nod factors, which initiate symbiotic responses on the roots of legume hosts and are required for recognition, controlled infection and nodule formation (DÉNARIÉ, et al., Annu Rev Biochem, vol.65, p.503, 1996; LEROUGE et al., Nature, vol.344, p.781, 1990; GEURTS and BISSELING, Plant Cell, vol.14, Suppl, S239, 2002). At pico-nano molar concentrations these molecules induce a variety of responses including rapid calcium influx and calcium spiking, specific gene induction, alterations in epidermal cell morphology and cortical cell mitosis (GEURTS and BISSELING, 2002, précité).

Genetic studies in the model legumes *Medicago truncatula* and *Lotus japonicus* have led to the identification of genes involved in the perception and transduction of Nod factors. Recently genes coding for two types of transmembrane receptor-like serine/threonine kinases with putative extracellular regions containing LysM domains (LysM-RLKs), required for Nod factor responses and rhizobial infection, have been cloned (RADUTOIU et al., Nature, vol.425, p.585, 2003; LIMPENS et al., Science, vol.302, p.630, 2003; MADSEN et al., Nature, vol.425, p.637, 2003). The gene products are hypothesized to form heterodimeric Nod factor receptors (RADUTOIU *et al.,* 2003, précité).

Downstream of these genes, three genes have been mapped in *M*. *truncatula, DMI1, DMI2* and *DMI3* that are required for both nodulation and the formation of arbuscular mycorrhizae (the AM symbiosis). Mutants in *DMI1, DMI2* and *DMI3* are blocked for most responses to Nod factors: They have a similar phenotype and do not exhibit induction of root hair branching (but instead form hair swelling), early nodulin gene expression and cortical cell division (CATOIRA et al., Plant Cell, vol.12, p.1647, 2000). However, they differ in their ability to respond to Nod factors by the induction of sharp oscillations of concentration of cytoplasmic calcium (calcium spiking), which is lost in *dmi1* and *dmi2* but not in *dmi3* mutants (EHRHARDT et al., Cell, vol.85, p.673, 1996; WAIS et al., Proc Natl Acad Sci U S A, vol.97, p.13407, 2000). Furthermore, mutants in *DMI1* and *DMI2 (= NORK)* genes, which are unable to form nodules and mycorrhizae (CATOIRA *et al.,* 2000, précité), are defective for most responses to Nod factors, including the calcium spiking response, but are still able to generate the rapid calcium influx response (EHRHARDT *et al.,* 1996, précité; SHAW and LONG, Plant Physiol, vol.131, p.976, 2003).

Recently, *DMI2* was shown to encode a LRR receptor-like protein kinase (NORK; ENDRE et al., Nature, vol.417, p.962, 2002).

The inventors have now isolated and characterized the *DMI3* gene of *M*. *truncatula,* and have found that it encodes a member of the small plant family of chimeric Ca²⁺-calmodulin-dependent protein kinases. They have also identified the orthologous *DMI3* gene in pea.

Members of the CCaMK group have been described in a number of plants ranging from the moss *Physcomitrella* to higher plants, both monocotyledonous and dicotyledonous. DMI3 is highly similar to rice, tobacco, and lily CCaMKs (73.5 % identity between *Medicago* and lily CCaMK). All these proteins share with the more common plant calcium-dependent protein kinases (CDPK), an N-terminal kinase domain. However they differs from CDPKs by the structure of their C-terminal calcium-binding regulatory domain that is more similar to a mammalian visinin-like domain (with three calcium-binding EF hands) than to the calmodulin-like domain typical of CDPKs (with four EF hands). Between the kinase and calcium-binding domains lies a calmodulin-binding domain that overlaps an autoregulatory domain. This structure allows regulation of the kinase activity by both calcium and calmodulin (SATHYANARAYANAN et al., J Biol Chem, vol.276, p.32940, 2001; TAKEZAWA et al., J Biol Chem, vol.271, p.8126, 1996).

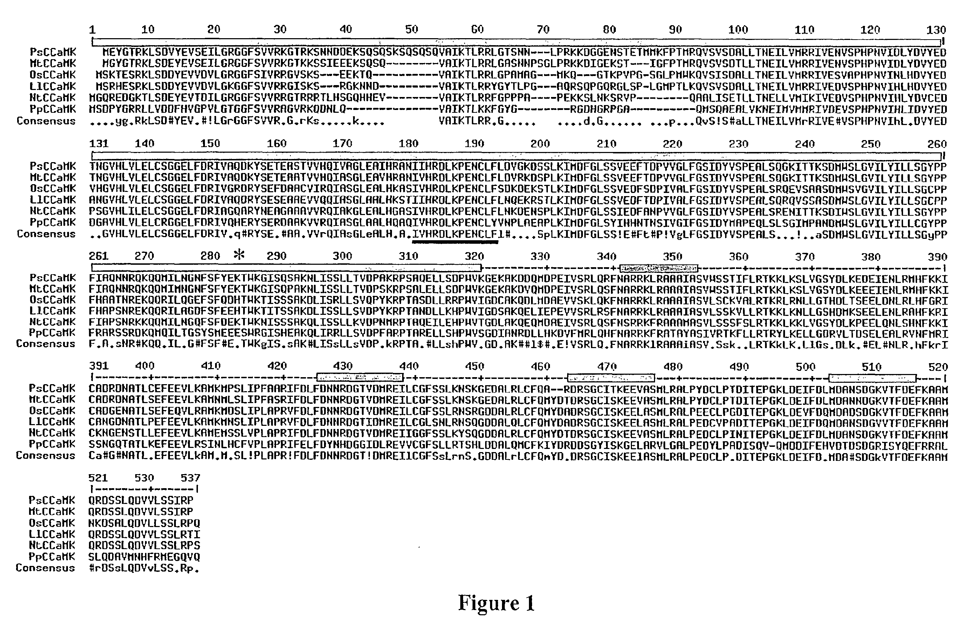

Figure 1 shows the comparison of CCaMKs of *M. truncatula* (MtCCaMK) and *P. sativum* (PsCCaMK) characterized by the inventors to previously characterized CCaMKs from lily (LlCCaMK : U24188), rice (OsCCaMK : AK070533), tobacco (NtCCaMK : AF087813) and *Physcomitrella* (PpCCaMK : AY155462). The serine/threonine kinase domain ( 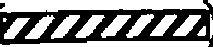 ), calmodulin-binding site ( 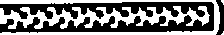 ), calcium-binding EF hand motifs ( 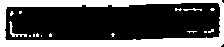 ) and autophosphorylation site (*) of CCaMKs are indicated above the sequences. The kinase active site signature ( 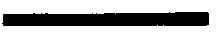 ) is indicated under the sequences. A putative bipartite nuclear localization signal can be detected between positions 60 and 75 in *M. truncatula* CCaMK sequence, but it is not present in the *P. sativum* sequence.

Little is known about the biological role of CCaMKs in plants. The fact that the lily, tobacco and rice CCaMKs are preferentially expressed in developing anthers and root tips (POOVAIAH et al., Planta, vol.209, p.161, 1999; WANG and POOVAIAH, J Biol Chem, vol.274, p.12001, 1999) has led to the suggestion that they could play a role in mitosis and meiosis (YANG and POOVAIAH, Trends Plant Sci, vol. 8, p.505, 2003).

The inventors show here for the first time, through the use of *DMI3* mutants, that a CCaMK plays a role in the signaling pathway leading to endomycorrhization and in the developmental process of nodulation in legumes. *DMI3* appears to act immediately downstream of calcium spiking and upstream of all other symbiotic responses. Without being bound to any particular mechanism, it may be hypothesised that the calcium spiking, and not the rapid calcium influx, is the calcium signature recognized by DMI3, the role of which would be to translate this signal to various cellular components controlling nodulation and mycorrhization responses.

The findings of the present invention allow to presume that previously known CCaMKs from non-leguminous plants, whose function was unknown until now, also play a part in endomycorrhization in said plants, and thus that modulating the expression or activity of said CCaMKs may allow to regulate endomycorrhization.

The present invention provides means to regulate the nodulation and/or endomycorrhization responses of plants to microbial symbionts.

The invention thus provides a method for regulating nodulation and/or endomycorrhization in a plant wherein said method comprises modulating the expression or activity of a CCaMK in said plant.

According to a preferred embodiment of the invention, said CCaMK has at least 80% identity or at least 90% similarity, preferably at least 85% identity or at least 90% similarity, more preferably at least 90% identity or at least 95% similarity, and still more preferably at least 95% identity or at least 99% similarity, with the polypeptide SEQ ID NO: 2

Unless otherwise specified, the sequence identity or similarity values provided herein are calculated using the BLAST programs (ALTSCHUL et al., Nucleic Acids Res., vol.25, p.3389, 1997) under default parameters, and the whole length of the reference sequence as a comparison window.

According to the invention, the expression of a CCaMK in a plant can be increased or decreased for the purpose of modulating nodulation and/or endomycorrhization.

The expression of a CCaMK in a plant can be increased, for instance, by expressing in said plant a DNA construct producing said polypeptide, or by altering the endogenous promoter of the *CCaMK* gene in order to up-regulate gene expression; conversely, a decrease of the expression of a CCaMK can be obtained for instance by expressing in said plant a polynucleotide inducing the silencing of a *CCaMK* gene, for instance by antisense inhibition or cosuppression. It is also possible to use ribozymes targeting CCaMK mRNA, or to alter the *CCaMK* promoter in order to down-regulate gene expression.

The CCaMK activity in a plant can be increased, for instance by expressing a mutant CCaMK devoid of the autoinhibitory domain located around the calmodulin binding domain, such as the mutant disclosed by RAMACHANDIRAN et al. (J. Biochem. (Tokyo), 121, 984-90, 1997). Conversely, the CCaMK activity in a plant can be decreased, for instance, by expressing a CCaMK having a mutation impairing the function of the kinase domain.

Preferably, said CCaMK is selected among the *Medicago truncatula* CCaMK of SEQ ID NO: 2 and the *Pisum sativum* CCaMK of SEQ ID NO: 4.

SEQ ID NO: 1 is a genomic DNA sequence encoding the *Medicago truncatula* CCaMK of SEQ ID NO: 2, and SEQ ID NO: 3 is a genomic DNA sequence encoding the the *Pisum sativum* CCaMK of SEQ ID NO: 4.

Other polynucleotides encoding a CCaMK can be obtained by methods well known in the art, such as screening genomic or cDNA libraries from any leguminous plant species able to form root nodules with rhizobia, using nucleic acid probes and/or primers obtained from SEQ ID NO: 1, 3 or degenerate probes or primers obtained from SEQ ID NO: 2 or 4.

By way of example, such polynucleotides can be obtained from genomic or cDNA libraries of Papilionoideae, in particular those belonging to any of the genus *Pisum, Glycine, Phaseol us,* or *Medicago.*

A polynucleotide encoding a CCaMK can be inserted into an expression cassette, under control of regulatory sequences which will direct the transcription and translation of the polynucleotide in an intended host cell.

Typically, said regulatory sequences comprise at least a promoter that drives expression in the host cell. They generally also comprise a transcription initiation start site, a ribosome binding site, an RNA processing signal, a transcription termination site, and/or a polyadenylation signal.

All or part of said regulatory sequences can be derived from the natural CCaMK gene, or consist of heterologous regulatory sequences.

By way of example, one may use the endogenous promoter of the CCaMK gene eventually altered in order to up-regulate or down-regulate gene expression; one may also replace the endogenous CCaMK promoter by an heterologous promoter providing a stronger or weaker level of expression, and/or a different expression profile (i.e a promoter conferring inducible or constitutive, environmentally or developmentally-regulated, or cell or tissue-specific/selective expression).

A large choice of promoters suitable for expression in varied hosts is available in the art.

In particular, promoters capable to direct transcription in plant cells include a large variety of promoters that can be obtained for instance from plants, plant viruses, or bacteria such as *Agrobacterium.* They include constitutive promoters, i.e. promoters which are active in most tissues and cells and under most environmental conditions, tissue or cell specific promoters which are active only or mainly in certain tissues or certain cell types, and inducible promoters that are activated by physical or chemical stimuli.

Non-limitative examples of promoters that are commonly used in plant cells are the cauliflower mosaic virus (CaMV) 35S promoter, the Nos promoter, the rubisco promoter. Advantageously, one may use a root-specific or root-preferred promoter.

Non limitative examples of root-specific or root-preferred promoters include the maize allothionein promoter (de FRAMOND et al., FEBS 290, 103-106, 1991); EP 452269), the acid chitinase promoter described by SAMAC et al., (Plant Physiol. 93, 907-914, 1990), the soybean root-specific glutamine synthetase promoter described by HIRE et al. (Plant Mol. Biol. 20, 207-218, 1992), or the root specific promoters described in WO/9113992 or US 5,837,848.

The expression cassette can be inserted into a host vector, by the conventional techniques of genetic engineering. The host vector may be any vector suitable for transfection of an intended host cell. By way of non-limitative example, vectors suitable for transfection of plant cells and/or for the establishment of transgenic plants can be derived from the tumor-inducing (Ti) plasmid of *Agrobacterium tumefaciens.*

These vectors may also comprise one or several marker gene(s) which confers a selectable phenotype on the host cells. Example of marker genes are reporter genes such as genes coding for beta-glucuronidase (GUS), for green fluorescent protein (GFP), for luciferase. Other examples of marker genes are antibiotic resistance genes, such as resistance to kanamycin, G418, bleomycin, hygromycin, or herbicide resistance genes, such as resistance to Basta.

Introduction of a polynucleotide encoding a CCaMK into host cells or tissues can be effected by means well known in the art, for instance by transfection, microinjection, electroporation, transduction, ballistic introduction, or the like. In the case of plant cells, one can also use *Agrobacterium rhizogenes* or advantageously, *Agrobacterium tumefaciens* mediated transformation.

The transformed cells, tissues, or organs can be used to regenerate transgenic plants. Many methods of cell transformation and plant regeneration for numerous plant species are known in the art.

The invention also provides means for evaluating the nodulation and/or endomycorrhization characteristics of a plant, in particular a legume.

In particular, the invention provides:
- a method for identifying an allele of a *CCaMK* gene associated with given nodulation and/or endomycorrhization characteristics, wherein said method comprises isolating a nucleic acid fragment comprising the *CCaMK* gene or a portion thereof from at least one plant expressing said phenotype and sequencing said fragment;

The invention further provides:
- a method for identifying a polymorphism in the *CCaMK* gene associated with nodulation and/or endomycorrhization phenotype, wherein said method comprises identifying, as described above, at least two different alleles of *CCaMK* associated with different nodulation and/or endomycorrhization phenotypes and comparing the sequences of said alleles.

Once a polymorphism has been identified, reagents and kits allowing the routine detection of said polymorphism can be designed. Commonly used reagents are nucleic acid probes, or restriction enzymes, or PCR primers, or combinations thereof. The choice of a reagent or of a combination of reagents depends of the nature of the polymorphism.

Preferred kits and reagents are those comprising a set of primers allowing specific PCR amplification of a DNA segment spanning the polymorphic locus. For microsatellites and insertion/deletion polymorphisms, PCR primers may be sufficient, since the allelic forms of the polymorphism may be differentiated by the size of the amplification product. In the case of single nucleotide polymorphisms (SNP), one will generally also use a restriction enzyme, allowing to differentiate the allelic forms by the presence or size of restriction fragments.

The invention also provides a method for testing a plant for its nodulation and/or endomycorrhization characteristics, wherein said method comprises detecting whether an allele of a *CCaMK* gene associated with given nodulation and/or endomycorrhization characteristics is present in said plant.

According to a preferred embodiment of the method of the invention, said nodulation and/or endomycorrhization characteristics are the lack of nodulation and/or endomycorrhization, or a reduced nodulation and/or endomycorrhization and the allele of the *CCaMK* gene associated with said phenotype expresses no CCaMK*,* or a lower level of CCaMK, or a less active CCaMK than the wild type allele, or an inactive CCaMK.

Examples of alleles resulting in said phenotype are:
- alleles having a deletion of all or part of the *CCaMK* gene, or that are splicing variants of the *CCaMK* gene, and result in the expression of a truncated CCaMK;
- alleles having a mutation resulting in the expression of a less active or inactive variant of CCaMK ;
- alleles having a mutation in the *CCaMK* promoter, resulting in a lower expression of CCaMK:

By way of example, some alleles of *Medicago truncatula* and *Pisum sativum* CCaMKs found by the inventors to be associated with said phenotype in are listed in Table I below:

**Table 1**

| Genetic background | Mutation |
|---|---|
| *Medicago truncatula* | 14 bp deletion: 198-203/stop |
| | CGA/TGA : R97/stop |
| *Pisum sativum* | CAA/TAA : Q230/stop |
| | CAA/TAA : Q230/stop |
| | CAA/TAA : Q230/stop |
| | TGG/TGA : W240/stop |
| | TGT/TGA : S224/stop |
| | CTT/C-T : L188/stop |
| | GGG/AGG : G202/R |
| | TCT/TTT: S24/F |

All these mutations are located in the kinase domain.

The present invention will be further illustrated by the additional description which follows, which refers to examples of obtention and use of polynucleotides encoding a CCaMK of the invention. It should be understood however that these examples are given only by way of illustration of the invention and do not constitute in any way a limitation thereof.

### EXAMPLE 1: CLONING AND CHARACTERIZATION OF M. TRUNCATULA DMI3.

The *DMI3* gene was amplified from genomic DNA of *M. truncatula* wild-type Jemalong A17 (CATOIRA *et al.,* 2000, précité) and of two *dmi3* mutants, TRV25 (SAGAN et al., in Biological nitrogen fixation for the 21st century Elmerich, Kondorosi, Newton, Ed, Kluwer Academic Publishers, p.317-318, 1998) and T1-5, both defective for nodulation. T1-5 mutant was obtained from a population of EMS mutagenized seeds, deriving from Jemalong A17 *ENOD11-GUS* (JOURNET et al., Mol Plant Microbe Interact, vol.14, p.737, 2001), by screening seedlings for the lack of GUS activity in roots after treatment with Nod factors.

The PCR primers used for amplification are listed in table II below:

**TABLE II**

| Sequence | Primer Name | Position in SEQ ID NO: 1: |
|---|---|---|
| GGCCAAATGTTGAACAGTGA | cand1A (SEQ ID NO:5) | 2819-2839 |
| GGGTTTTGATTGCTACTTGTGA | cand1B (SEQ ID NO:6) | 3288-3315 |
| TCACCATGGGATATGGAACA | cand2A (SEQ ID NO:7) | 3166-3185 |
| CCACAGTTGCAGCTTCAGTC | cand2B (SEQ ID NO:8) | 3605-3624 |
| CTTTGTTCCGGTGGTGAACT | cand3A (SEQ ID NO:9) | 3549-3568 |
| TGGACATGTTGAGTTTGACGA | cand3B (SEQ ID NO:10) | 4050-4070 |
| TCGTGGTTTTAACAAAACTCCA | cand4A (SEQ ID NO:11) | 3990-4011 |
| ATTTTGGGCAATGAAAGGTG | cand4B (SEQ ID NO:12) | 4473-4491 |
| TTTCGTTAAACTCAACGTATCCAA | cand5A (SEQ ID NO:13) | 4387-4410 |
| CATTTGAAGATAATGTCATGAAAAA | cand5B (SEQ ID NO:14) | 4908-4932 |
| TGACTTAAAGGACCAATTTATAACAA | cand6A (SEQ ID NO:15) | 4819-4844 |
| TCGACATTTTTGCCAGTTGA | cand6B (SEQ ID NO:16) | 5383-5399 |
| AAATTTCACAAGGAACCAAACA | cand7A (SEQ ID NO:17) | 5285-5306 |
| GAAGCAAAAGGGATCAATGATAA | cand7B (SEQ ID NO:18) | 5828-5850 |
| TGTGCAGATAGAGACAATGCAA | cand8A (SEQ ID NO:19) | 5765-5786 |
| CAAGTTATGAGGCCCCAATG | cand8B (SEQ ID NO:20) | 6203-6222 |
| CAACATTGGGGCCTCATAAC | cand9A (SEQ ID NO:21) | 6200-6219 |
| CAATCATATGGCAAAGCCTGT | cand9B (SEQ ID NO:22) | 6765-6785 |
| ATCCATGCTCAGGGTAATGC | cand10A (SEQ ID NO:23) | 6674-6693 |
| ACGACGATCACACTCACAGG | cand10B (SEQ ID NO:24) | 7204-7223 |

| | | |
|---|---|---|
| PCR amplification were performed using the Taq polymerase (IN VITROGEN) according to the manufacturer instructions (MgCl2 2,5 mmol/1). PCR program: *1 cycle (94° for 5 min) * 30 cycles (94° for 15 s, 55° for 15 s, 72° for 1 min) * 1 cycle (72° for 7 min). | | |

The genomic sequence of the wild-type *DMI3* is represented in the annexed sequence listing under SEQ ID NO:1.

The *DMI3* gene comprises 7 exons. The full length open reading frame of 1,569 nucleotides codes for a protein of 523 amino-acids with a predicted molecular mass of 58,600 Da.

The position of the introns and the exons in the sequence SEQ ID NO:1 is as follows:
exon:3171-3901
exon2: 4467-4521
exon3: 4600-4710
exon4 : 5000-5232
exon5 : 5764-5971
exon6 : 6627-6686
exon7 : 6769-6942

Analysis of the deduced polypeptide sequence indicated that DMI3 belongs to the calcium and calmodulin-dependent protein kinase group of serine-threonine protein kinases, together with proteins encoded by genes previously characterized from tobacco, lily, rice and the moss *Physcomitrella* (Figure 1).

Like these proteins, DMI3 shares with the more common plant calcium-dependent protein kinases (CDPK), an N-terminal kinase domain. However it differs from CDPKS by the structure of its C-terminal calcium-binding regulatory domain that is more similar to a mammalian visinin-like domain (with three calcium-binding EF hands) than to the calmodulin-like domain typical of CDPKs (with four EF hands). Between the kinase and calcium-binding domains lies a calmodulin-binding domain that overlaps an autoregulatory domain. This structure allows regulation of the kinase activity by both calcium and calmodulin.

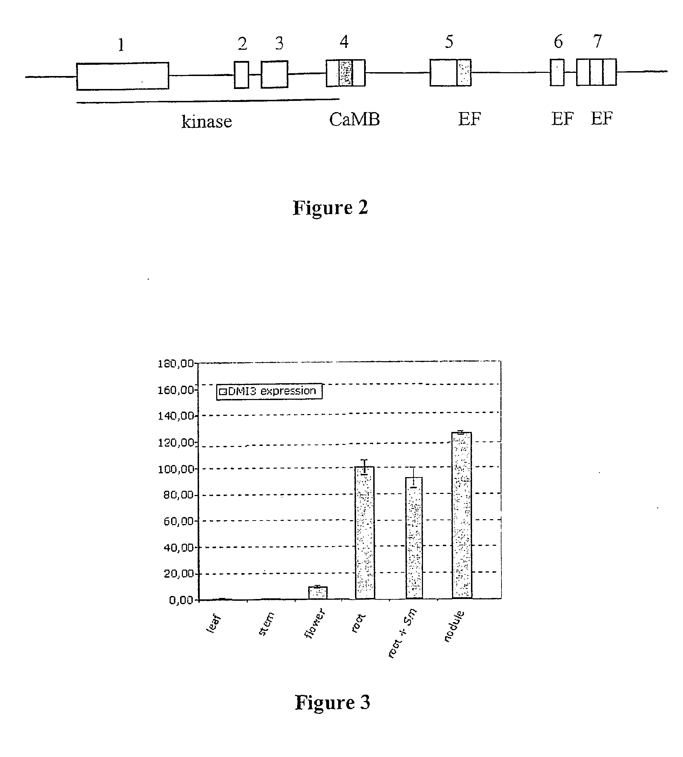

The intron-exon structure of the *DMI3* gene and the position of the predicted protein domains and motifs are schematized in Figure 2.
The kinase domain ( 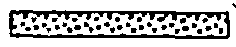 ), calmodulin binding (CaMB) domain ( 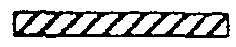 ) and calcium-binding EF hands ( 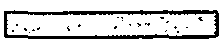 ) are indicated.

### EXAMPLE 2: FUNCTIONAL COMPLEMENTATION OF A DMI3 MUTATION BY THE WILD-TYPE CCAMK CLONE.

Roots of seedlings of the TRV25 *dmi*3 mutant, defective for nodulation (SAGAN *et al.,* 1998, précité), were transformed with *Agrobacterium rhizogenes* strain Arqual harboring the pF5 plasmid which carries the wild-type *DMI3* gene.

A 11 kb *XbaI* fragment, carrying the wild type *DMI3* coding sequence and 1,040 bp upstream of the start codon, was cloned into pCAMBIA2201 (www.cambia.org). The resulting pF5 recombinant plasmid was introduced into A. *rhizogenes* Arqual by electroporation (MCCORMAC et al., Molecular Biotechnology, vol.9, p.155, 1998). Hairy root transformation of TRV25 *dmi3* mutant was carried out as described (BOISSON-DERNIER et al., Mol Plant Microbe Interact, vol.14, p.695, 2001), with the following modifications. Transgenic roots were first selected on Fahraeus agar medium supplemented with kanamycin (20 mg 1-1), then the transformed seedlings were transferred to pouches and inoculated with the rhizobial symbiont *Sinorhizobium meliloti* GMI6526 = RCR2011 (pXLGD4), which carries a *lacZ* reporter gene to facilitate the in *planta* visualization of the bacteria.

Nodules were scored two weeks after inoculation. Experiments were performed twice. 90 % of plants exhibited transformed roots as detected by GUS activity. In total, 45 plants transformed by pF5 were tested, 36 of them nodulated with an average of 5 nodules per plant. A X-Gal treatment and microscopic examination of a representative sample of nodules revealed the presence of bacteria inside the nodules. No nodule could be detected on 26 control plants transformed by the pCAMBIA2201 vector.

This complementation together with the genetic evidence described in Example 1, demonstrate that the *DMI3* encodes a CCaMK required for nodulation and mycorrhization phenotype.

### EXAMPLE 3: EXPRESSION PATTERN OF DMI3 MRNA IN M. TRUNCATULA.

*DMI3* mRNA levels were measured by quantitative RT-PCR in leaves, stems, flowers, roots, roots 48h post-inoculation with *S*. *meliloti,* and root-nodules of *M*. *truncatula,* and normalized against *AtACTIN2 (MtACT2)* that is constitutively expressed in all tissues tested.

Total RNA was extracted and treated by DNAseI. cDNA was synthesized from 1 µg total RNA using the Taqman Gold RT-PCR kit (Perkin-Elmer Applied Biosystems) in a total volume of 50 µl. Quantitative RT-PCR reactions were performed in triplicate on 6.5 µl cDNA using the SYBR-GreenR PCR Master kit (Perkin-Elmer Applied Biosystems) (40 cycles of 95°C for 10 s, 58°C for 1 min) and real-time detection was performed on the ABI 7700 and analyzed using the GeneAmp 5700 SDS software (Perkin-Elmer Applied Biosystems).

Primers used:
DMI3-1: TCATTGATCCCTTTTGCTTCTCGT (SEQ ID NO:25),
DMI3-2: GATGCTACTTCCTCTTTGCTGATGC (SEQ ID NO:26),
MtACT2: TGGCATCACTCAGTACCTTTCAACAG (SEQ ID NO:27),
MtACT2-2: ACCCAAAGCATCAAATAATAAGTCAACC (SEQ ID NO:28).

The results are shown in Figure 3. A 10-fold higher expression of *DMI3* was observed in roots compared to flowers, whereas no significant expression was detected in leaves or stems. Further, a slight upregulation of the root expression of *DMI3* in roots as well as in root nodules, in response to the symbiotic partner *S*. *meliloti,* could be observed 48 h post inoculation.

The *DMI2 (=NORK)* gene, another *M. truncatula* gene required for nodulation and mycorrhization, whose mutation results in a symbiotic phenotype similar to that of the *dmi3* mutant TRV25, apart from calcium spiking, was studied in parallel. Its pattern of expression was similar to that of *DMI3,* although the level of expression was higher for *DMI2* (data not shown).

### EXAMPLE 4: CLONING AND ISOLATION OF A DMI3 HOMOLOGUE IN PEA.

Mutants with phenotypes and syntenic map positions similar to that of TRV25 have been described in pea. As *dmi3* mutants, pea mutants at the *sym9* locus are altered immediately downstream of the calcium spiking response induced by treatment of root hairs by Nod factors, and are defective for root hair curling, infection thread formation, nodulation, and endomycorrhization.

PCR amplification was performed from genomic DNA of wild-type and *sym9* mutants of *P*. *sativum.* The plants are listed in Table III below:

**Table III**

| Variety | | Reference |
|---|---|---|
| Frisson | Wild type | ( DUC and MESSAGER, Plant Science, vol.60, p.207, 1989) |
| P1 | EMS *sym9* mutant of Frisson | ( DUC and MESSAGER, 1989, précité) |
| P2 | EMS *sym9* mutant of Frisson | ( DUC and MESSAGER, 1989, précité) |
| P3 | EMS *sym9* mutant of Frisson | ( DUC and MESSAGER, 1989, précité) |
| P53 | EMS *sym9* mutant of Frisson | ( SAGAN et al., Plant Science, vol.100, p.59, 1994) |

The primers used, designed on the basis of the *M. truncatula DMI3* sequence, are listed in Table IV below:

**TABLE IV**

| Sequence | PRIMER NAME | POSITION IN SEQ ID NO: 1 |
|---|---|---|
| GGTGGCGAACTTTTCGATAG | pscand1 (SEQ ID NO:29) | 705-724 |
| ACCTGAAAAACAATCAAAGCAA | pscand2 (SEQ ID NO:30) | 1199-1220 |
| GCAGTTGACTCTCTCTTTGTCC | pscand3 (SEQ ID NO:31) | 1-14 |
| TCTGTGCTATTTTCACCACCA | pscand4 (SEQ ID NO:32) | 506-526 |
| AACGAGAGAGGACTACAATAGACGA | pscand6 (SEQ ID NO:33) | 1104-1128 |
| TCAAAATCACAGCTGGAACC | pscand7 (SEQ ID NO:34) | 1928-1947 |
| GGAAGAGGTGGATTTTCTGTTG | pscand8 (SEQ ID NO:35) | 348-369 |
| TCAGGCTTCAAATCCCTATGA | pscand9 (SEQ ID NO:36) | 821-841 |
| AAAATTGAAATCCTTGGTTGGA | pscand10 (SEQ ID NO:37) | 1696-1717 |
| AACACAAACGGAGAGCATCC | pscand11 (SEQ ID NO:38) | 2161-2180 |
| AGAAAATCGTGAGGCGGATT | pscand12 (SEQ ID NO:39) | 1887-1906 |
| TTGGTGATGCATCCTGATCT | pscand13 (SEQ ID NO:40) | 2282-2305 |
| TTGTGGCTTTTCGAGTCTCA | pscand14 (SEQ ID NO:41) | 2125-2144 |
| GCAGCTTTGAATTCATCAAATGTA | pscand15 (SEQ ID NO:42) | 2511-2534 |
| TGGACGAATAGAAGAGAGAACTACA | pscand16 (SEQ ID NO:43) | 2562- |

| | | |
|---|---|---|
| PCR amplification were performed using the Taq polymerase (IN VITROGEN) according to the manufacturer instructions (MgCl2 2,5 mmol/1). PCR program: * 1 cycle (94° for 5 min) * 30 cycles (94° for 15 s, 55° for 15 s, 72° for 1 min) * 1 cycle (72° for 7 min). | | |

The genomic sequence obtained from wild-type *P. sativum* cv Frisson is represented in the annexed sequence listing under SEQ ID NO:3.

The gene comprises 7 exons. It encodes a CCaMK with high overall similarity (90 % identity) to that of *M*. *truncatula.*

The position of the introns and the exons in the sequence SEQ ID NO: 2 is as follows:
exon1: 294-1048
exon2: 1219-1273
exon3: 1349-1459
exon4: 1541-1773
exon5: 1978-2192
exon6: 2284-2330
exon7: 2416-stop

Each sequenced CCaMK from eight *sym9* mutants of *P. sativum,* obtained in three different genetic backgrounds, exhibited single base-pair alterations in the kinase domain predicted to alter the CCaMK function. This further supports a role of CCaMK in nodulation and mycorrhization. In addition, it allows the molecular characterization of a major gene controlling both nodulation and mycorrhization in pea, an important grain legume crop.

Various combinations of the following primers designed on the basis of the *M*. *truncatula DMI3* sequence were used for PCR amplification of *P. sativum SYM9* genomic DNA in the different wild-type cultivars and *sym9* mutants.

## Claims

1. A method for regulating nodulation and/or endomycorrhization in a plant wherein said method comprises modulating the expression or activity in said plant of a Ca²⁺-calmodulin-dependent protein kinase (CCaMK).

2. A method of claim 1, wherein said CCaMK has an amino acid sequence having at least 80% identity or at least 90% similarity, with the polypeptide SEQ ID NO: 2.

3. The method of claim 2, wherein said CCaMK is selected among the *Medicago truncatula* CCaMK of SEQ ID NO: 2 and the *Pisum sativum* CCaMK of SEQ ID NO: 4.

4. The method of any of claims 1 to 3, wherein the expression or activity of said CCaMK in said plant is increased for enhancing nodulation and/or endomycorrhization.

5. The method of any of claims 1 to 3, wherein the expression or activity of said CCaMK in said plant is decreased for reducing nodulation and/or endomycorrhization.

6. A method for identifying an allele of a *CCaMK* gene, said allele being associated with a given phenotype of nodulation and/or endomycorrhization characteristics, wherein said method comprises isolating a nucleic acid fragment comprising the *CCaMK* gene or a portion thereof, from at least one plant expressing said phenotype, and sequencing said fragment.

7. A method for testing a plant for its nodulation and/or endomycorrhization characteristics, wherein said method comprises detecting whether an allele of a *CCaMK* gene associated with given nodulation and/or endomycorrhization characteristics is present in said plant.

## Patentansprüche

1. Verfahren zum Regulieren einer Knöllchenbildung und/oder Endomykorrhisierung in einer Pflanze, wobei das Verfahren ein Modulieren der Expression oder der Aktivität in der Pflanze von einer Ca²⁺-calmodulinabhängigen Proteinkinase (CCaMK) beinhaltet.

2. Verfahren nach Anspruch 1, wobei die CCaMK eine Aminosäuresequenz mit zumindest 80 % Übereinstimmung oder zumindest 90 % Ähnlichkeit mit der Polypeptid-SEQ-ID-NO: 2 aufweist.

3. Verfahren nach Anspruch 2, wobei die CCaMK aus der *Medicago-truncatula-*CCaMK der SEQ-ID-NO: 2 und der *Pisum-sativum-CCaMK* der SEQ-ID-NO: 4 ausgewählt wird.

4. Verfahren nach einem der Ansprüche 1 bis 3, wobei die Expression oder die Aktivität der CCaMK in der Pflanze zur Verbesserung der Knöllchenbildung und/oder Endomykorrhisierung erhöht wird.

5. Verfahren nach einem der Ansprüche 1 bis 3, wobei die Expression oder die Aktivität der CCaMK in der Pflanze verringert wird, um eine Knöllchenbildung und/oder Endomykorrhisierung zu reduzieren.

6. Verfahren zum Identifizieren eines Allels eines *CCaMK-Gens,* wobei das Allel einem bestimmten Phänotyp von Knöllchenbildungs- und/oder Endomykorrhisierungseigenschaften zugeordnet wird, wobei das Verfahren ein Isolieren eines Nucleinsäurefragments, das das *CCaMK-Gen* oder einen Teil desselben aufweist, aus zumindest einer Pflanze, die diesen Phänotyp exprimiert, und ein Sequenzieren des Fragmentes beinhaltet.

7. Verfahren zum Testen einer Pflanze in Bezug auf deren Knöllchenbildungs- und/oder Endomykorrhisierungseigenschaften, wobei das Verfahren ein Erfassen dessen beinhaltet, ob ein Allel eines *CCaMK-Gens,* das bestimmten Knöllchenbildungs- und/oder Endomykorrhisierungseigenschaften zugeordnet ist, in der Pflanze vorhanden ist.

## Revendications

1. Procédé de régulation de la nodulation et/ou de l'endomycorhization dans une plante, dans lequel ledit procédé comprend la modulation de l'expression ou de l'activité dans ladite plante d'une protéine kinase dépendante de la calmoduline - Ca²⁺ (CCaMK).

2. Procédé selon la revendication 1, dans lequel ladite CCaMK a une séquence d'acide aminé ayant au moins 80% d'identité ou au moins 90% de similarité, avec le polypeptide de la SEQ. ID N°2.

3. Procédé selon la revendication 2, dans lequel ladite CCaMK est choisie parmi la CCaMK *Medicago truncatula* de la SEQ. ID N°2 et la CCaMK *Pisum sativum* de la SEQ. ID N°4.

4. Procédé selon l'une quelconque des revendications 1 à 3, dans lequel l'expression ou l'activité de ladite CCaMK dans ladite plante est accrue pour augmenter la nodulation et/ou l'endomycorhization.

5. Procédé selon l'une quelconque des revendications 1 à 3, dans lequel l'expression ou l'activité de ladite CCaMK dans ladite plante est diminuée pour réduire la nodulation et/ou l'endomycorhization.

6. Procédé pour identifier un allèle d'un gène *CCaMK,* ledit allèle étant associé à un phénotype donné de caractéristiques de nodulation et/ou d'endomycorhization, dans lequel ledit procédé comprend l'isolation d'un fragment d'acide nucléique comprenant le gène *CCaMK* ou une partie de celui-ci, à partir d'au moins une plante exprimant ledit phénotype, et le séquençage dudit fragment.

7. Procédé de test d'une plante pour ses caractéristiques de nodulation et/ou d'endomycorhization, dans lequel ledit procédé comprend la détection de la présence ou non dans ladite plante d'un allèle d'un gène *CCaMK* associé à une nodulation et/ou d'endomycorhization donnée.
